# EUROPÄISCHE PATENTSCHRIFT

(11) **EP 1 257 521 B1**
(45) Veröffentlichungstag und Bekanntmachung des Hinweises auf die Patenterteilung: **15.06.2005**
(21) Anmeldenummer: 01919297.0
(22) Anmeldetag: 16.02.2001
(51) Int. Cl.: C07C 209/16, C07C 213/04, C07C 231/02

(54) **VERFAHREN ZUR HERSTELLUNG VON AMINEN AUS EPOXIDIERTEN OLEFINEN**
METHOD FOR THE PRODUCTION OF AMINES FROM EPOXIDISED OLEFINS
PROCEDE POUR PRODUIRE DES AMINES A PARTIR D'OLEFINES EPOXYDEES

(30) Priorität: 18.02.2000 DE 10007554
(43) Veröffentlichungstag der Anmeldung: 20.11.2002
(73) Patentinhaber: BASF AKTIENGESELLSCHAFT, 67056 Ludwigshafen (DE)
(72) Erfinder: OTT, Christian, 67346 Speyer (DE); BERGEMANN, Marco, 68766 Hockenheim (DE)
(74) Vertreter: Isenbruck, Günter, Dr.
(86) Internationale Anmeldenummer: PCT/EP2001/001783
(87) Internationale Veröffentlichungsnummer: WO 2001/060782

(56) Entgegenhaltungen:
- WO-A-97/44366
- US-A- 4 159 996

## Beschreibung

Die Erfindung betrifft ein Verfahren zur Herstellung von Aminen aus epoxidierten Olefinen.

Amine sind wichtige Zwischenprodukte für die chemische und pharmazeutische Industrie. Amine werden üblicherweise durch Umsetzung von Alkylhalogeniden mit Ammoniak, durch Anlagerung von Ammoniak an olefinische Doppelbindungen, durch aminierende Hydrierung von Aldehyden und Ketonen, durch katalytische Hydrierung von Carbonsäurenitrilen sowie durch katalytische Reduktion von Nitroalkanen mit Wasserstoff hergestellt, siehe Ullmann's Encyclopädie der Technischen Chemie, 4. Auflage 1974, Band 7, Seite 375.

In WO 97/44 366 ist ein Verfahren zur Herstellung von Polyalkenaminen durch Umsetzung eines Polyalkenepoxids mit einem Amin und Dehydratisierung und Reduktion des gebildeten Aminoalkohols zum Polyalkenamin beschrieben. Die eingesetzten Polyalkenepoxide leiten sich von Polyalkenen mit einem zahlenmittleren Molekulargewicht von mindestens etwa 175 bis 40000 ab. Als bevorzugtes Polyalken ist Polyisobuten mit einem zahlenmittleren Molekulargewicht von etwa 200 bis 3000 genannt.

US 4,159,996 offenbart ein Verfahren zur Herstellung von 1,2-Diaminen durch Reaktion von vicinalen Nitroketonen mit dem Hydrochlorid eines primären Amins und anschließender katalytischer Hydrierung der gebildeten Nitroalkylimino- beziehungsweise Nitrooximinoalkane zu den Diaminen.

Aufgabe der Erfindung ist, ein Verfahren zur Herstellung von niedermolekularen 1,2-Diaminen aus epoxidierten niedermolekularen Olefinen bereitzustellen.

Gelöst wird die Aufgabe durch ein Verfahren zur Herstellung von Aminen, bei dem ein Epoxid der Formel (I) 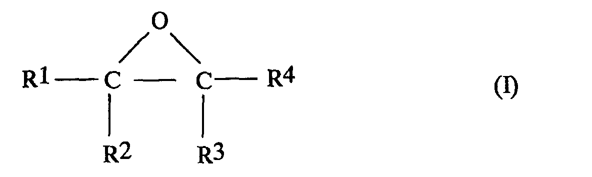 worin R², R³ und R⁴ Wasserstoff und R¹ einen linearen oder verzweigten, gesättigten oder ungesättigten C₂-C₂₀₀-Kohlenwasserstoffrest bedeuten,
mit Ammoniak und Wasserstoff in Gegenwart eines Katalysators umgesetzt wird,
wobei das molare Verhältnis Ammoniak:Epoxid von 5:1 bis 500:1 und der Wasserstoffdruck von 10 bar absolut bis 500 bar absolut beträgt.

R¹ bedeutet einen linearen oder verzweigten, gesättigten oder ungesättigten C₂-C₂₀₀-Kohlenwasserstoffrest. Bevorzugt sind gesättigte Kohlenwasserstoffreste, insbesondere lineare gesättigte Kohlenwasserstoffreste. Von den genannten Kohlenwasserstoffresten sind C₂-C₅₀-Kohlenwasserstoffreste bevorzugt, insbesondere die C₂-C₃₀-Kohlenwasserstoffreste. Beispiele sind n-Ethyl, n-Propyl, n-Butyl, n-Pentyl, n-Hexyl, n-Heptyl, n-Octyl, n-Nonyl und n-Decyl.

Die Epoxide der Formel (I) werden aus den entsprechenden Olefinen durch Epoxidation gewonnen. Die Epoxidation wird dabei beispielsweise so durchgeführt, daß man das Olefin in einem geeigneten Lösungsmittel, beispielsweise Diethylether, oder anderen dipolar aprotischen Lösungsmitteln oder apolaren Lösungsmitteln, wie Xylol oder Toluol, löst, diese Lösung gegebenenfalls trocknet, das Epoxidationsmittel zusetzt und gegebenenfalls unter leichtem Erwärmen, zum Beispiel auf etwa 30 bis 100°C, epoxidiert. Epoxidationsmittel sind beispielsweise Persäuren, wie Perameisensäure, Peroxibenzoesäure, m-Chlorperoxibenzoesäure oder Peroxiessigsäure sowie die aus H₂O₂ und den entsprechenden Carbonsäuren gebildeten Persäuren, oder Alkylperoxide, wie tert.-Butylhydroperoxid, wobei m-Chlorperbenzoesäure und Peroxiessigsäure bevorzugt sind.

In dem erfindungsgemäßen Verfahren bedeutet R¹ einen Kohlenwasserstoffrest und bedeuten R², R³ und R⁴ jeweils ein Wasserstoffatom. Bevorzugte Reste R¹ sind C₂-C₅₀-, besonders bevorzugt C₂-C₃₀-Kohlenwasserstoffreste. Von diesen sind die linearen, insbesondere die linearen gesättigten Kohlenwasserstoffreste besonders bevorzugt. Olefinepoxide, von denen nach dieser Ausführungsform des erfindungsgemäßen Verfahrens ausgegangen wird, sind beispielsweise 1-Butenepoxid, 1-Hexenepoxid, 1-Octenepoxid, 1-Decenepoxid, 1-Dodecenepoxid, 1-Tetradodecenepoxid, 1-Hexadecenepoxid, 1-Octadecenepoxid und Rübölmethylester-Epoxid. Das Hauptprodukt der Umsetzung ist ein 1,2-Diamin der Formel (II)

HR¹(NH₂)C-C(NH₂)H₂ (II)

Gemäß einer bevorzugten Ausführungsform erfolgt die Umsetzung des Epoxids (I) zum Amin einstufig, in dem man das Epoxid (I) mit Ammoniak in Gegenwart von Wasserstoff und einem Katalysator umsetzt, der Dehydratisierung- und gleichzeitig Hydrierungseigenschaften aufweist. Diese einstufige Umsetzung wird vorzugsweise zur Herstellung der 1,2-Diamine (II) durchgeführt.

Der erfindungsgemäß verwendbare Katalysator mit Dehydratisierungs- und Hydrierungseigenschaften ist vorzugsweise ausgewählt unter Zeolithen oder porösen Oxiden von Al, Si, Ti, Zr, Nb, Mg und/oder Zn, sauren Ionenaustauschern und Heteropolysäuren, welche jeweils mindestens ein Hydriermetall tragen. Als Hydriermetall verwendet man bevorzugt Ni, Co, Cu, Fe, Pd, Pt, Ru, Rh oder Kombinationen davon.

Erfindungsgemäß brauchbare Zeolithe sind beispielsweise saure zeolithische Feststoffkatalysatoren, die beschrieben sind in der EP 0 539 821, worauf hiermit Bezug genommen wird. Als Beispiele für geeignete Zeolithe sind zu nennen Zeolithe mit Mordenit-, Chabasit- oder Faujasit-Struktur; Zeolithe vom Typ A, L, X und Y; Zeolithe vom Pentasiltyp mit MFI-Struktur; Zeolithe, in denen Aluminium und/oder Silizium ganz oder teilweise durch Fremdatome ersetzt sind, z.B. Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische oder Titansilikatzeolithe, wie TS-1, ETS 4 und ETS 10.

Zur Optimierung von Selektivität, Umsatz und Standzeiten können die erfindungsgemäß verwendeten Zeolithe in geeigneter Weise mit weiteren Elementen dotiert werden, wie dies z.B. in der EP 0 539 821 beschrieben ist.

In gleicher Weise kann eine Dotierung der Zeolithe mit den oben genannten Hydriermetallen erfolgen. Das Hydriermetall sollte, berechnet als Oxid, in einem Anteil von 1 bis 10 Gew.-%, bezogen auf das Gesamtgewicht der katalytischen aktiven Masse, enthalten sein,

Weitere geeignete Katalysatoren mit Dehydratisierungs- und Hydrierungs-eigenschaften sind vorzugsweise saure Oxide der Elemente Al, Si, Zr, Nb, Mg oder Zn oder Gemische davon, die mit wenigstens einem der oben genannten Hydriermetalle dotiert sind. Das Oxid (berechnet als Al₂O₃, SiO₂, Nb₂O₅, MgO oder ZnO) ist dabei in einem Anteil von etwa 10 bis 99 Gew.-%, vorzugsweise etwa 40 bis 70 Gew.-%, in der Katalysatormasse (d.h. katalytisch aktiven Masse) enthalten. Das Hydriermetall (berechnet als NiO, CoO, CuO, Fe₂O₃, PdO, PtO, RuO₂ oder Rh₂O₃) ist dabei in einem Anteil von etwa 1 bis 90 Gew.-%, vorzugsweise etwa 30 bis 60 Gew.-%, bezogen auf das Gesamtgewicht der Katalysatormasse, enthalten. Außerdem können in den erfindungsgemäß eingesetzten Oxiden geringe Mengen, d.h. etwa 0,1 bis etwa 5 Gew.-% (berechnet für die Oxide) weiterer Elemente, wie z.B. Mo oder Na enthalten sein, um Katalysatoreigenschaften, wie Selektivität und Standzeit, zu verbessern.

Oxide dieses Typs und deren Herstellung sind beispielsweise beschrieben in der EP 0 696 572, worauf hiermit Bezug genommen wird. Die Herstellung erfolgt vorzugsweise dadurch, daß man eine wässrige Salzlösung herstellt, welche die oben genannten Katalysator-Komponenten enthält, und durch Zugabe einer Mineralbase, wie z.B. Natriumcarbonat, gegebenenfalls unter leichtem Erwärmen, copräzipitiert. Den Niederschlag trennt man ab, wäscht, trocknet und calziniert, wie z.B. durch 4-stündiges Erwärmen auf 500°C.

Die oben beschriebenen erfindungsgemäßen Zeolithe und aktiven Oxide können gegebenenfalls konditioniert werden, indem man sie gegebenenfalls auf eine bestimmte Korngröße vermahlt und zu Strängen oder Tabletten presst, wobei Formhilfsmittel, wie z.B. Graphit, zugesetzt werden können.

Erfindungsgemäß besonders bevorzugt ist die Verwendung eines Katalysators, der, bezogen auf das Gesamtgewicht der katalytisch aktiven Masse,
etwa 30 Gew.-% Zr, berechnet als ZrO₂,
etwa 50 Gew.-% Ni, berechnet als NiO,
etwa 18 Gew.-% Cu, berechnet als CuO,
etwa 1,5 Gew.-% Mo, berechnet als MoO₃ und
etwa 0,5 Gew.-% Na, berechnet als Na₂O
enthält.

Alkoxylierungskatalysatoren, welche dem Reaktionsgemisch erfindungsgemäß vorzugsweise zugesetzt werden, fördern die Öffnung des Epoxid-Ringes. Beispiele für geeignete Alkoxylierungskatalysatoren sind Wasser und Alkohole, wie Methanol, Ethanol, Mineralsäuren und Carbonsäuren.

Die Epoxide werden in beiden der oben beschriebenen Verfahrensvarianten, die sowohl kontinuierlich als auch diskontinuierlich durchgeführt werden können, bei einer Reaktionstemperatur von etwa 100 bis 300°C, vorzugsweise etwa 150 bis 250°C, insbesondere etwa 180 bis 220°C und einem Wasserstoffdruck von im allgemeinen 10 bar bis 500 bar, bevorzugt 50 bar bis 300 bar, insbesondere etwa 250 bar umgesetzt. Ammoniak wird, bezogen auf das Epoxid in einem molaren Verhältnis von 5:1 bis 500:1, bevorzugt 10:1 bis 500:1, besonders bevorzugt 20:1 bis 250:1 eingesetzt. Die Reaktionszeit beträgt im allgemeinen 1 Stunde bis 48 Stunden, bevorzugt 1 Stunde bis 24 Stunden, besonders bevorzugt 4 Stunden bis 24 Stunden, insbesondere etwa 12 Stunden. Die Umsetzung kann sowohl in Substanz als auch in Gegenwart eines Lösungsmittels, beispielsweise Kohlenwasserstoffe wie Hexan, oder THF durchgeführt werden, bevorzugt wird sie so durchgeführt. Bevorzugtes Lösungsmittel ist THF.

Das erfindungsgemäße Verfahren zeichnet sich durch hohe Umsätze und eine hohe Selektivität aus. So beträgt bei der Herstellung von 1,2-Diamin (II) aus 1-Olefinepoxiden, wie 1-Epoxidecan, 1-Epoxidodecan, 1-Epoxitetradecan und 1-Epoxihexadecan, die Ausbeute an 1,2-Diamin mindestens 80 Mol.-%, vorzugsweise mindestens 85 Mol.-%, besonders bevorzugt mindestens 90 Mol.-%. Als Nebenprodukte werden in geringem Maße 1-Monoamine, sekundäre Amine, sekundäre Aminoalkohole sowie Piperazine gebildet.

Die Erfindung wird durch die nachstehenden Beispiele näher erläutert.

### Beispiele 1-5:

150 g des Epoxids werden mit 100 g Katalysator H1/88 in 800 ml THF im Autoklaven vorgelegt. Es wird 2 mal mit 5 bar Stickstoff inertisiert, anschließend werden bei Raumtemperatur 500 ml Ammoniak aufgepresst. Anschließend werden bei Raumtemperatur zunächst 50 bar Wasserstoff aufgepresst und es wird auf 200°C hochgeheizt. Bei Erreichen der Temperatur von 200°C wird weiterer Wasserstoff bis 250 bar aufgepresst. Es wird 12 Stunden bei 200°C gerührt. Dann wird auf 30°C abgekühlt und der Reaktor vorsichtig entspannt. Es wird auf 40°C erneut erwärmt und noch eine Stunde lang gerührt. Schließlich wird mit 10 bar Stickstoff gespült und auf Raumtemperatur abgekühlt. Der Reaktorinhalt wird filtriert und einrotiert.

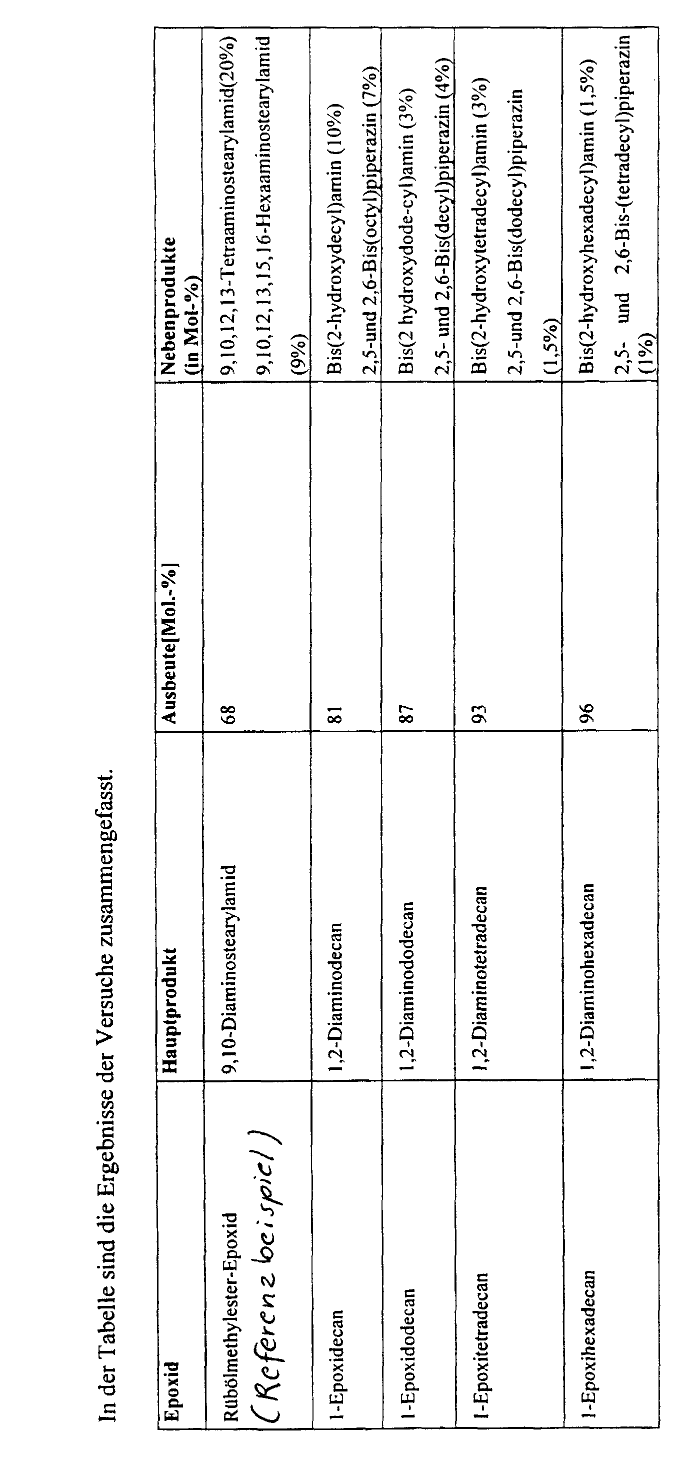

## Patentansprüche

1. Verfahren zur Herstellung von Aminen, bei dem ein Epoxid der Formel (I) 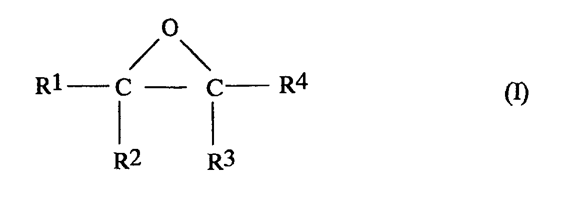 worin R¹, R², R³ und R⁴ unabhängig voneinander Wasserstoff oder einen linearen oder verzweigten, gesättigten oder ungesättigten C₂-C₂₀₀-Kohlenwasserstoffrest bedeuten mit Ammoniak und Wasserstoff in Gegenwart eines Katalysators umgesetzt wird, wobei das molare Verhältnis Ammoniak:Epoxid von 5:1 bis 500:1 und der Wasserstoffdruck von 10 bar bis 500 bar beträgt, **dadurch gekennzeichnet, dass** R¹ einen Kohlenwasserstoffrest und R², R³ und R⁴ ein Wasserstoffatom bedeuten und das Hauptprodukt der Umsetzung ein 1,2-Diamin der Formel (II)
HR¹(NH₂)C-C(NH₂)H₂ (II)
ist.

2. Verfahren nach Anspruch 1, **dadurch gekennzeichnet, daß** das Verfahren einstufig durchgeführt wird.

3. Verfahren nach Anspruch 1 oder 2, **dadurch gekennzeichnet, daß** der Kohlenwasserstoffrest ein C₂-C₃₀-Kohlenwasserstoffrest ist.

4. Verfahren nach einem der Ansprüche 1 bis 3, **dadurch gekennzeichnet, daß** der Kohlenwasserstoffrest ein linearer Alkylrest ist.

5. Verfahren nach einem der Ansprüche 1 bis 4, **dadurch gekennzeichnet**, das die Reaktionstemperatur 100 bis 300°C beträgt.

6. Verfahren nach einem der Ansprüche 1 bis 5, **dadurch gekennzeichnet, daß** die Umsetzung in THF als Lösungsmittel durchgeführt wird.

## Claims

1. A process for preparing amines, which comprises reacting an epoxide of the formula (I) 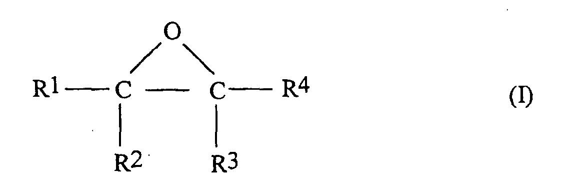 where R¹, R², R³ and R⁴ are each, independently of one another, hydrogen or a linear or branched, saturated or unsaturated C₂-C₂₀₀-hydrocarbon radical, with ammonia and hydrogen in the presence of a catalyst, where the molar ratio of ammonia:epoxide is from 5:1 to 500:1 and the hydrogen pressure is from 10 bar to 500 bar, wherein R¹ is a hydrocarbon radical and R², R³ and R⁴ are each a hydrogen atom and the main product of the reaction is a 1,2-diamine of the formula (II)
HR¹(NH₂)C-C(NH₂)H₂ (II).

2. A process as claimed in claim 1 carried out in a single step.

3. A process as claimed in claim 1 or 2, wherein the hydrocarbon radical is a C₂-C₃₀-hydrocarbon radical.

4. A process as claimed in any of claims 1 to 3, wherein the hydrocarbon radical is a linear alkyl radical.

5. A process as claimed in any of claims 1 to 4, wherein the reaction temperature is from 100 to 300°C.

6. A process as claimed in any of claims 1 to 5, wherein the reaction is carried out in THF as solvent.

## Revendications

1. Procédé de préparation d'amines, dans lequel on fait réagir un époxyde de la formule (I) : 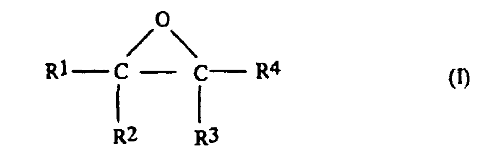 dans laquelle R¹, R², R³ et R⁴ représentent indépendamment l'un de l'autre de l'hydrogène ou un radical d'hydrocarbure en C₂-C₂₀₀ linéaire ou ramifié, saturé ou insaturé, avec de l'ammoniac et de l'hydrogène, en présence d'un catalyseur, le rapport molaire ammoniac/époxyde étant de 5/1 à 500/1 et la pression d'hydrogène de 10 bars à 500 bars, **caractérisé en ce que** R¹ représente un radical d'hydrocarbure et R², R³ et R⁴ un atome d'hydrogène et le produit principal de la réaction est une 1,2-diamine de la formule (II) :
HR¹(NH₂)C-C(NH₂)H₂ (II)

2. Procédé suivant la revendication 1, **caractérisé en ce que** le procédé est effectué en une étape.

3. Procédé suivant la revendication 1 ou 2, **caractérisé en ce que** le radical d'hydrocarbure est un radical d'hydrocarbure en C₂-C₃₀.

4. Procédé suivant l'une des revendications 1 à 3, **caractérisé en ce que** le radical d'hydrocarbure est un radical alkyle linéaire.

5. Procédé suivant l'une des revendications 1 à 4, **caractérisé en ce que** la température réactionnelle est de 100 à 300°C.

6. Procédé suivant l'une des revendications 1 à 5, **caractérisé en ce que** la réaction est effectuée dans du THF comme solvant.
